# EUROPEAN PATENT SPECIFICATION

(11) **EP 2 197 417 B1**
(45) Date of publication and mention of the grant of the patent: **14.03.2012**
(21) Application number: 08839029.9
(22) Date of filing: 16.10.2008
(51) Int. Cl.: A61K 9/00, A61K 9/06, A61K 47/36

(54) **PHARMACEUTICAL COMPOSITION COMPRISING S-NITROSOGLUTATHIONE AND POLYSACCHARIDE**
PHARMAZEUTISCHE ZUSAMMENSETZUNG MIT S-NITROSOGLUTATHION UND POLYSACCHARID
COMPOSITION PHARMACEUTIQUE COMPRENANT DE LA S-NITROSOGLUTATHIONE ET UN POLYSACCHARIDE

(30) Priority: 17.10.2007 HU 0700678
(43) Date of publication of application: 23.06.2010
(73) Proprietor: PHARMAGENIX AG, 8001 Zurich (CH)
(72) Inventor: LACZA, Zsombor, 1121 Budapest (HU); HORNYÁK, István, 3530 Miskolc (HU)
(74) Representative: Török, Ferenc
(86) International application number: PCT/HU2008/000124
(87) International publication number: WO 2009/050527

(56) References cited:
- US-A1- 2002 002 136
- SEABRA AMEDEA BAROZZI ET AL: "Solid films of blended poly(vinyl alcohol)/poly(vinyl pyrrolidone) for topical S-nitrosoglutathione and nitric oxide release." May 2005 (2005-05), JOURNAL OF PHARMACEUTICAL SCIENCES MAY 2005, VOL. 94, NR. 5, PAGE(S) 994 - 1003 , XP008079458 ISSN: 0022-3549 cited in the application page 995, column 1, lines 7-16 page 996, column 1, lines 15-26 page 996, column 1, line 44 - column 2, line 18 page 1001, column 2, lines 6-32
- SEABRA A B ET AL: "S-Nitrosoglutathione incorporated in poly(ethylene glycol) matrix: potential use for topical nitric oxide delivery" NITRIC OXIDE: BIOLOGY AND CHEMISTRY, ACADEMIC PRESS, vol. 11, no. 3, 1 November 2004 (2004-11-01), pages 263-272, XP004654783 ISSN: 1089-8603 cited in the application

## Description

### Field of the invention

The invention primarily relates to pharmaceutical compositions comprising S-nitrosoglutathione (GSNO) and one or more polysaccharide-type polymer(s) together with one or more pharmaceutically accepted polymer(s) and additive(s). The invention is based on the discovery that polysaccharide-type polymers (such as chitosan) are capable of stabilizing the otherwise highly labile GSNO.

### State of the art

Vasoconstriction that develops during microcirculatory disturbances, increasing susceptibility to thrombosis and accumulation of free radicals having released in certain metabolic problems cause complex tissue damage that leads to a decrease in wound healing potential and to chronic ulceration in several cases [Greenman et al., 2005, Lancet, 366, 1711-7; Sigaudo-Roussel et al., 2004, Diabetes, 53, 1564-9; Veves et al., 1998, Diabetes, 47, 457-63; Hile and Veves, 2003, Curr. Diab. Rep., 3, 446-51; Nikolovska et al., 2005, Acta Dermatovenerol Croat, 13, 242-6]. Numerous dermatological medicaments are available for treating microcirculatory disturbances developing in, e.g., diabetes or vasoconstriction. Characteristically, these compositions contain essential oils (e.g. rosemary) and other non specific active ingredients having clinically unverified efficiency. According to experimental results, nitric oxide (NO) can beneficially affect the microcirculatory disturbance [Cals-Grierson and Ormerod, 2004, Nitric Oxide, 10, 179-93]. NO is a quickly reacting gaseous compound, having - among others - smooth muscle relaxing effect, which is used as an inhalant in phyhisiotherapy. Due to its consistency, NO can hardly be used for dermatological purposes, however, clinical observation confirms its effectiveness for treating non-healing ulcers [Miller et al., 2004, J. Cutan. Med. Surg., 8, 233-8]. Alternatively, by using NO donor compounds, such as sodium nitroprusside, therapeutically effective amounts of NO can be transferred to the epidermis, however, the administration of these compounds is accompanied by several new problems that make their application difficult. Namely, most of the NO donors release not only NO, but also other reactive nitrogen species which can be harmful for the tissues during long term application. A more important problem comes from the fact that the degradation of NO donors is very fast, accordingly blood-stream increasing compositions having suitable stability and predictable local vasodilator effect are not known. Thirdly, by absorbing through the skin, the slowly degrading NO donor compositions reach the systemic circulation and exert their effect in tissues far from the treated area, which is not preferable.

Certain scientific studies have already been directed to using the substrate of NO synthase, i.e. L-arginine, in the treatment of microcirculatory disturbances [Fossel, 2004, Diabetes Care, 27, 284-5]. The NO synthase system itself is necessary for the L-arginine to exert its activity, however, the damage of this enzyme system is characteristic for the microcirculatory disturbance. Additionally, the L-arginine is also a substrate of different other NO-synthase-competing enzymes, such as argininase, arginine decarboxylase etc. Thus, on the basis of the above, obviously it is more preferable to administer the NO to the local circulatory system, than the application of its precursor.

Nitrate-containing skin patches are widely used in medicine, and their effects are partly based on their NO-donor feature. However, the nitrate considered as a precursor of the vasodilator agent (NO) is transferred to the systemic circulation without the increase in blood circulation of the directly exposed skin surface. The desired effect of a NO-donor for treating microcirculatory disturbance is just the opposite to it: it should generate local vasodilation without exerting significant systemic effects.

Numerous patents are known where NO-donor compound is used in topical compositions releasing nitrogen monoxide in a desired speed. For example, the US 6,287,601 B1 patent discloses a formulation in which nitroglycerine, hydroxilamine, nitroprusside, nitrate or azide are used as NO-donor compounds in combination with a non-steroidal anti-inflammatory drug (NSAID). The US 5,519,020 patent discloses the use of a water-insoluble nitrogen oxide/polymer adduct, where the polymer might be, e.g., polyethyleneimine cellulose. The US 7,048,951 B1 B2 patent discloses that powdered sodium nitrite, ascorbic acid and preferably maleic acid are mixed, and the obtained mixture releases nitrogen monoxide when exposed to water.

Several embodiments are known where a polymer-based matrix comprises nitrogen monoxide bound physically or chemically. The US 5,994,444 patent discloses that a biologically degradable polymer (preferably poly-L-lactic acid) is impregnated with nitrogen oxid donor compound, preferably with an inorganic nitrite compound. The US 5,770,645 2 patent discloses polymers which are derivatized with -NOx group which is then able to release NO.

The NOlabs (Helsinborg, Sweden) submitted several applications (W02006/084911-14) where nitrogen monoxide is used for the treatment of different diseases, including diabetic ulcer and neuropathy. In these diseases NO releasing polymers are used to obtain the desired NO release. Preferably, an NO-derivative of linear polyethyleneimine (L-PEI-NO) is used. In the general disclosure the chitosan is referred to as a type of certain polymers that can be derivatized with NO. Furthermore, the polysaccharides are referred to only as an inert support for the NO-releasing polymers (e.g. WO 2006/084912, pp. 11-12).

Research studies verify that an endogenous nitrosothiol compound, the GSNO, which is a natural NO-donor, is particularly suitable for the preparation of local vasodilator composition [Sogo et al., 2000, Br. J. Pharmacol., 131, 1236-44]. NO and reduced glutathione with known antioxidant effect are generated during the decomposition of GSNO. Due to its vasodilatory and platelet aggregation inhibitory effects, the NO penetrated into the local circulation improves blood circulation in the skin and inhibits the thrombus formation [Khan et al., 2005, J. Cereb. Blood Flow Metab., 25, 177-92; Kuo et al., 2004, J. Surg. Res., 119, 92-9; Sogo et al., 2000, Biochem. Biophys. Res. Commun., 279, 412-9]. However, its applicability is limited since in aqueous solutions the half-life of this compound is very short, only 5.5 hours.

The stability of GSNO could be significantly improved by using pharmaceutically known vehicles. Poly(ethyleneglycol), poly(vinyl-pyrrolidone), or poly(vinyl-alcohol) are all suitable to decrease the degradation rate of GSNO, primarily by forming hydrogen bridges [A. B. Seabra et al., May 2005, J. Pharm. Sci., 95, No.5, 994-1003; A. B. Seabra, M.G de Oliveira, 2004, Biomaterials, 25, 3773-82; Seabra et al., 2004, Nitric Oxide, 11, 263-72]. However, these approaches are not sufficient to generate stabile composition appropriate for everyday medical practice, since the half-life of the agent at ambient temperature or at 4°C could be extended only for a few days.

US 2002/0002136 discloses compositions of glutathione (GSH,GSSH) or nitrosoglutathione or glutathione monoalkyl ester with a polycation such as chitosan.

The role of stablizing hydrogene bridges is also emphasized in the US 7,015,347 B2 patent, where compounds having intramolecular OH or SH goups capable of stabilizing the S-NO group were claimed.

Additionally, NO-donor macromolecules containing S-NO groups covalently bound to polyethylene glycol framework are also known [Seabra et al., 2005 Spt-Oct, Biomacromolecules, 6(5), 2512-20].

Only one publication discloses that GSNO comprising hydrogels were administered to healthy volunteers and NO-dependent increase in local blood-stream was observed in the study [Seabra et al., 2004, Bristish J. Dermalol, 151, 977-83]. The rate of vasodilation correlated well with both the applied concentration of GSNO and the metabolic NO-products measured in the skin, thus verifying the specificity of the effect. No side effects were reported by the subjects during the study. In the study poly(ethyleneoxide)/poly(propyleneoxide) based Synperonic F127 hydrogels (Uniquema, Belgium) were used as vehicles. However, hydrogel composition used in the study is not suitable for clinical application, since it does not decelerate the decomposition of GSNO, hence it should be prepared freshly for each administration.

Nitrosoglutathione derivatives developed for local vasodilation are also known [see Lacer SA, Hungarian patent application No. P0105203]. These cyclic compounds have not been applied in the clinical practice so far, this is why there is no information about their efficiency or metabolism. Generally speaking, the endogenous agent with well-known metabolism, such as GSNO, has probably less side effect compared with synthetic derivatives, therefore it is more preferable.

The object of the invention is a vasodilator composition suitable for tiermatological application which is sufficiently stable under storage conditions in phamacy and household, in addition it can be easily applied and capable of causing clinically significant increase in blood-flow. Therefore, it can preferably be used for the treatment and the prevention of ulcer, neuropathy, such as diabetic peripheral neuropathy, and diabetic leg syndrome.

### Summary of the invention

In searching for the solution for the problems set forth above the inventors conducted extensive studies and discovered that the effect of polysaccharide(s), e.g. chitosan, can significantly decrease the metabolic rate of GSNO. Based on these unexpected results, the invention provides a composition comprising GSNO which remains suitably stable during storage, after being placed on the skin (preferably after regeneration) the composition exerts significant local vasodifative and blood-stream increasing effects, thus it can be used in the prevention and treatment of the above mentioned diseases.

The inventors discovered that by using polysaccharide(s), such as chitosan, the stability of such known GSNO-containing composition can be increased, in which one or more known pharmaceutically acceptable polymer may be used as vehicle with stabilizing effect, optionally together with additive(s). An especially unexpected observation is that in polysaccharide, preferably chitosan, containing polymer mixtures the GSNO shows higher stability than in the pure polymers themselves.

Accordingly, the present invention relates to a pharmaceutical composition characterized in that it comprises GSNO and a polysaccharide, together with PVA and PEG polymers and one or more pharmaceutically acceptable additive(s).

The referred embodiments are as follows:
Pharmaceutical composition in the form of an aqueous gel and containing PVA and PEG polymers as pharmaceutically acceptable polymer.
Pharmaceutical composition which is formulated in a lyophilized form.
Preferably chitosan is the polysaccharide in the above use.

### Detailed description of the invention

### Definitions

### Polysaccharide

The expression of "polysaccharide" refers to macromolecular carbohydrates where the monomers bind to each other through glycoside bonds (glycans). It includes important biopolymers, such as starch, glycogen and the cellulose (which can be regarded as polycondensation products of dextran and glucose), the inulin (polycondensation product of fructose), chitin, alginic acid etc. The above mentioned polysaccharids are the polycondensation products of saccharids of only a single type, hence they can be regarded as homopolymers. Naturally, in embodiments of the invention polysaccharids comprising different monomers (heteroglycans, e.g. hemicelluloses, heparin, hyaluric acid, murein) can also be used. Several derivatized polysaccharid variations are known (e.g. deacyled, sulfonized, etc. derivatives) which can also be used in the embodiment of the invention.

### Chitosan

A particularly referred polysaccharid is the chitosan (β-1,4-poly-D-glucoseamine) which can be considered as a deacylated derivative of the chitin (β-1,4-poly-N-acetyl-D-glucoseamine). Generally the chitosan is built up from more than 5000 glucoseamine units, thus its molecular weight can be even several million daltons. The chitosan is well known as a cholesterol lowering agent, can be applied as cellulose-like dietary fibres, it has a beneficial effect on the lipid levels, and it is recommended for preventing atherosclerosis and treating liver and kidney diseases. Additionally, it promotes wound healing and inhibits inflammatory processes [Azad et al., 2004, J. Biomed. Mater. Res. B. Appl. Biomater., 69, 216-22; Muzzarelli et al., 1999, EXS, 87, 251-64]. Furthermore, the body metabolizes the chitosan without harmful end-products, therefore the chitosan can also be used in body cavities [Khor and Lim, 2003, Biomaterials, 24, 2339-49].

### GSNO

The GSNO (S-nitrosoglutathione) is an endogenous compound having an important role in the metabolism of NO. The reduced glutathione as a free radical capturing tripeptide found in cells and certain cell components, such as mitochondria, is capable of reacting with NO which binds to the sulphur atom of the side-chain of the central tyrosine in the molecule and a nitrosoglutathione is formed. During the metabolism of GSNO this bond dissociates and the NO is released, thus the GSNO is not only a free radical capturing molecule but it is also an NO transporter molecule. A significant amount of GSNO can be found not only in the cells, but it is also present in the extracellular space, e.g. in the blood, thus its physiological function is the contribution in NO transport and in maintenance ol' constant NO blood level.

Several reaction scemes are known for synthesizing GSNO. According to a known reaction, sodium nitrite and later acetone is added to cold, acidic aqueous glutathione solution, preferably in multiple aliquots and during agitation. After the separation and the washing of the resulting precipitate, suitably pure S-nitrosoglutathione is obtained [Tetrahedron Letters, Vol. 26, No. 16, 2013-2016, 1985]. Other preparation methods are disclosed in Acc. Chem. Res. 1999, 32, 869-876;J. Chem. Soc. Perkin Trans. I., 1994, where the feasibility of conducting the reaction in acidic environment is also disclosed.

The GSNO is a brown colored compound having a characteristic absorption spectrum. One of its two characteristic peaks is in the UV range, while the maximum of the other is around 540 nm. During decomposition the absorption spectrum of GSNO goes through a change. The change in the height of the peak at 540 nm is linearly proportional with the concentration of GSNO. Wavelengths at far IR range can be used as background absorption, since no change occurs in them during the decomposition process of GSNO. These features allow monitoring the concentration of GSNO spectrophotometrically.

### Pharmaceutically acceptable polymers

The applied polysaccharide (preferably chitosan) significantly decreases the degradation of GSNO also in itself, however it is used together with other pharmaceutically acceptable polymer type compounds, poly(vinylalcohol) [PVA] and polyethylemglycol [PEG].

### Pharmaceutically acceptable additives

Furthermore, the composition may contain any such usual additive that is necessary for the optimalization of the physical features of the composition. Thus, it may contain inert vehicles, gelating agents, viscosity enhancers, colourants, buffering agents, odorants, preservatives, stabilizers etc.

The compositions according to the invention are preferably hydrogels or such dry compositions that can be transformed to hydrogels for use in medication by contacting them with water.

The hydrogel-type composition preferably contains distilled water or aqueous isotonic solution.

### Method for preparation

In the preparation of the composition according to the invention preferably an aqueous gel is prepared from the polysaccharide or from the polymer mixture used, then the GSNO is mixed into it in a desired concentration. If desired, the obtained gel is liophylised. For a long-time storage, it is feasible to keep the liophylised composition in a refrigerator. Feasibly, the liophylised composition is regenerated with water, preferably with distilled water, right before the application.

### Description of drawings

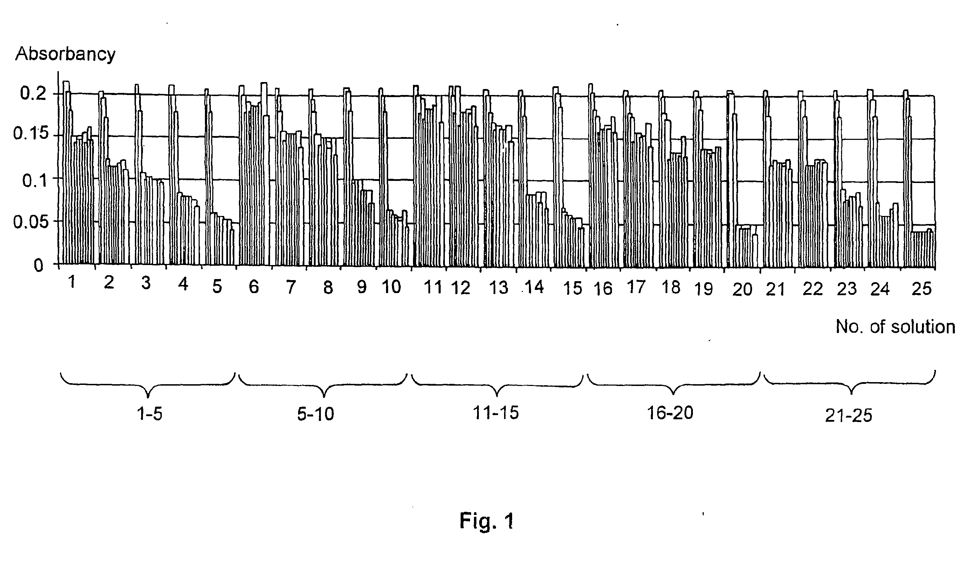

Figure 1 shows the results obtained in Example 3. On the X-axis the number of the solution and on the Y-axis the absorbancy values are given.

Degradation of each solution was monitored on days as follows: 0., 1., 2., 5., 6., 12., 15., 20., 21., 28., 35. and 44. Although regarding each solution series of 5 solutions the columns partially overlap, the shape of the decrease in absorbance of GSNO can be seen clearly within the studied 44-day time period for each solution series.

Figure 2 shows the results obtained in Example 4. On the X-axis the number of the solution and on the Y-axis the relative absorbancy values are given (the absorbancy of the starting solution is regarded as 100). Decomposition of each solution was observed on day 28.

Figure 3 shows the results obtained in Example 5. On the X-axis the number of the solution and on the Y-axis the relative absorbancy values are given (the absorbancy of the starting solution is regarded as 100). Decomposition of each solution was observed on day 69.

### Examples

The list of materials used in the examples are as follows:
1. Chitosan, technical purity (Sigma-Aldrich)
2. Chitosan, low molecular weight (Sigma-Aldrich)
3. Chitosan, medium molecular weight (Sigma-Aldrich)
4. L-glutathione (reduced, 99% purity, Sigma-Aldrich)
5. Sodium nitrite (99% purity, Sigma, Aldrich)
6. Polyethyleneglycol (average mol wt: 200; Sigma-Aldrich)
7. Poly(vinyl alcohol), 80% hydrolyzed, average mol wt: 9000 -10000 (Sigma-Aldrich)
8. Lactic acid (Fluka)
9. Analytically pure deionized water (Millipore Milli-Q)

### Measuring GNSO

The decomposition of GSNO was monitored spectrophotometrically, since the absorption spectrum of GSNO undergoes changes and the size change of the peak at 540 nm is linearly proportional with the concentration of GSNO. Wavelengths at far IR range were used as background absorption, since no change occurs in them during the decomposition of GSNO.

### Example 1

### Preparation of GSNO

### Method A

1.53 g (5 mmol) L-glutathione (GSH) was dissolved in a mixture of 5.5 ml water and 2.5 ml (2 N) aqueous HCl solution cooled in ice bath, then 0.345 g (mmol) sodium nitrite was added. The mixture was stirred for 40 min at 5°C, then 10 ml acetone was added and the solution was stirred for further 10 min. The precipitated brown deposit was filtered and subsequently washed with ice-cold water (5 x 1 ml), acetone (3 x 10 ml) and ether (3x10 ml). Thus 1.29 g (3.8 mmol) of S-nitrosoglutathione was obtained (76% yield).

### Method B

Firstly 0.204 g (0.666 mmol) GSH, then equimolar amount of NaNO₂ was added to 8 ml deionized water, and the mixture was kept on ice and stirred for further 10 min in dark. The calculated concentration of the obtained fresh solution is 2.726 w%.

In subsequent experiments freshly prepared GSNO solution according to above method B was used.

### Example 2

Previously prepared PVA and chitosan gels were mixed to the GSNO solution of example 1B in an amount diluting the original GSNO solution to 3-fold. 200 µl aliquots were pipetted into the wells of a 96-well plate in duplicates. The.plates were covered and stored at 4°C in dark. Since during storage the preparations lost different amounts of water, after finishing the experiment it became necessary to complete them with water to the original volume. GSNO concentration was expressed as the % decrease of optical density measured spectrophotometrically at the start and at the end of the experiment.

### Example 3

### [on the basis of HI11 measuring set]

### solutions 1-5:

Stock solution: 0.2 g PVA and 0.6 g PEG dissolved in 4 ml of water (Millipore Milli-Q). The following solutions were made from the stock:

### solutions 1-5:

1. 700 µl stock solution + 100 µl aqueous chitosan solution (1%)
2. 725 µl stock solution+ 75 µl aqueous chitosan solution (1%)
3. 750 µl stock solution+ 50 µl aqueous chitosan solution (1%)
4. 775 µl stock solution + 25 µl aqueous chitosan solution (1%)
5. 800 µl stock solution

### solutions 6-10:

Stock solution: 0.15 g PVA and 0.65 g PEG dissolved in 4 ml of water (Millipore Milli-Q). Solutions 6-10 were prepared from this according to the volumes given for solutions 1-5.

### solutions 11-15:

Stock solution: 0.1 g PVA and 0.7 g PEG dissolved in 4 ml of water (Millipore Milli-Q). Solutions 11-15 were prepared from this according to the volumes given for solutions 1-5.

### solutions 16-20:

Stock solution: 0.05 g PVA and 0.75 g PEG dissolved in 4 ml of water (Millipore Milli-Q). Solutions 16-20 were prepared from this according to the volumes given for solutions 1-5.

### solutions 21-25:

Stock solution: 0.8 g PEG dissolved in 4 ml of water (Millipore Milli-Q) (PVA-free solution). Solutions 20-25 were prepared from this according to the volumes given for solutions 1-5.

The experiments were performed analogously to example 2.

Figure 1 shows the results obtained. Degradation of each solution was monitored on days as follows: 0., 1.,2.,5.,6., 12., 15., 20., 21., 28., 35. and 44. Although regarding each solution series of 5 solutions the columns partially overlap, the shape of the decrease in absorbance of GSNO can be seen clearly within the studied 44-day time period for each solution series. It also can be seen that always the highest chitosan concentration shows the highest stability within the solution series. Interestingly, the best results were obtained with solution series 6-10 and 11-15, therefore the system is sensitive also to the PVA/PEG ratio.

### Example 4

Stock solution: 1 g PVA dissolved in 4 ml of water (Millipore Milli-Q) (PEG-free solution). The following solutions were prepared from the stock:

### solutions 1-4:

1. 400 µl stock solution+ 400 µl aqueous chitosan solution (1%)
2. 600 µl stock solution + 200 µl aqueous chitosan solution (1%)
3. 700 µl stock solution+ 100 µl aqueous chitosan solution (1%)
4. 800 µl stock solution (chitosane-free).

### solutions 5-8:

Stock solution: 0.8 g PVA and 0.2 g PEG dissolved in 4 ml of water (Millipore Milli-Q). Solutions 5-8 were prepared from this according to the volumes given for solutions 1-4.

### solutions 9-12:

Stock solution: 0.6 g PVA and 0.4 g PEG dissolved in 4 ml of water (Millipore Milli-Q). Solutions 9-12 were prepared from this according to the volumes given for solutions 1-4.

### solutions 13-16:

Stock solution: 0.4 g PVA and 0.6 g PEG dissolved in 4 ml of water (Millipore Milli-Q). Solutions 13-16 were prepared from this according to the volumes given for solutions 1-4.

### solutions 17-20:

Stock solution: 0.2 g PVA and 0.8 g PEG dissolved in 4 ml of water (Millipore Milli-Q). Solutions 17-20 were prepared from this according to the volumes given for solutions 1-4.

### solutions 21-24:

Stock solution: 1 g PEG dissolved in 4 ml of water (Millipore Milli-Q) (PVA-free solution). Solutions 21-24 were prepared from this according to the volumes given for solutions 1-4.

The experiments were performed analogously to example 2.

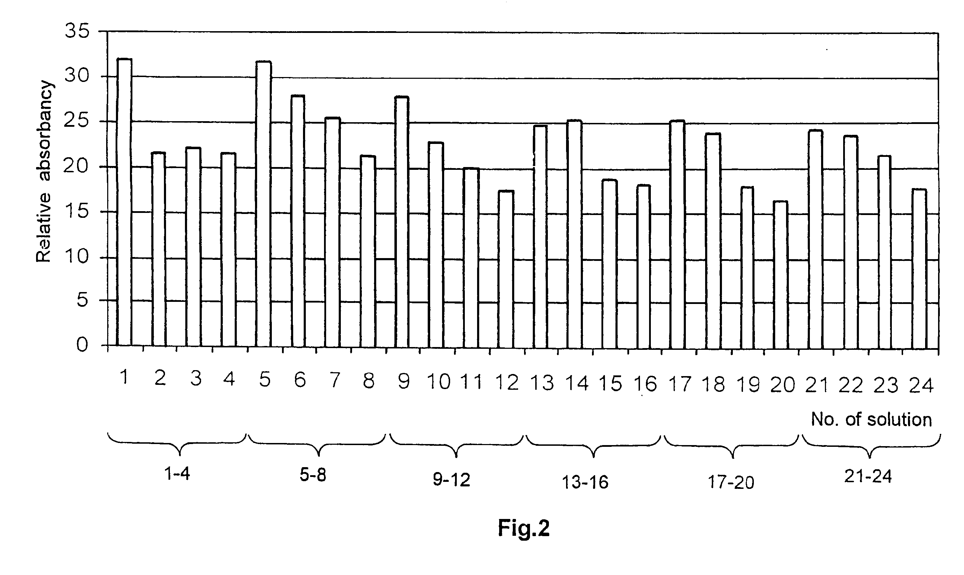

Figure 2 shows the results obtained. Decomposition of each solution was observed on day 28.

### Example 5

Stock solution: 0.8 g PVA dissolved in 4 ml of water (Millipore Milli-Q) (PEG-free solution). The following solutions were made from the stock:

### 1-4. solutions 1-4:

1. 400 µl stock solution+400 µl aqueous chitosan solution (1%)
2. 600 µl stock solution + 200 µl aqueous chitosan solution (1%)
3. 700 µl stock solution + 100 µl aqueous chitosan solution (1%)
4. 800 µl stock solution (chitosan-free).

### solutions 5-8:

Stock solution: 0.6 g PVA and 0.2 g PEG dissolved in 4 ml of water (Millipore Milli-Q). Solutions 5-8 were prepared from this according to the volumes given for solutions 1-4.

### solutions 9-12:

Stock solution: 0.4 g PVA and 0.4 g PEG dissolved in 4 ml of water (Millipore Milli-Q). Solutions 9-12 were prepared from this according to the volumes given for solutions 1-4.

### solutions 13-16:

Stock solution: 0.2 g PVA and 0.6 g PEG dissolved in 4 ml of water (Millipore Milli-Q). Solutions 13-16 were prepared from this according to the volumes given for solutions 1-4.

The experiments were performed analogously to example 2.

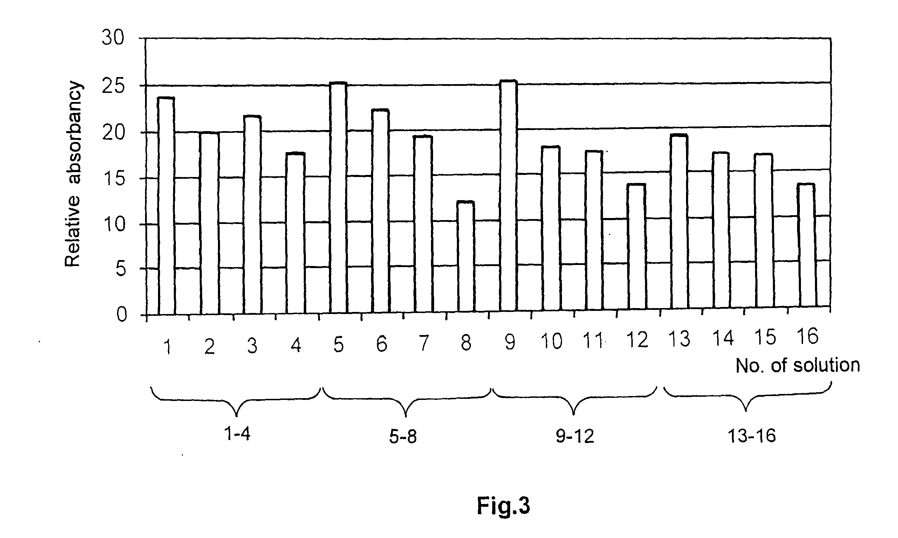

Figure 3 shows the results obtained. Decomposition of each solution was observed on day 69.

## Claims

1. Pharmaceutical composition **characterized in that** it comprises S-nitrosoglutathione (GSNO) and a polysaccharide, together with polyl(vinylalcohol) (PVA) and polyethyleneglycol (PEG) polymers and one or more pharmaceutically acceptable additive(s).

2. Pharmaceutical composition according to claim 1 **characterized in that** the polysaccharide is chitosan.

3. Pharmaceutical composition according to claim 1 or 2 **characterized in that** the composition is an aqueous gel.

4. Pharmaceutical composition according to any or claims 1-3 **characterized in that** the composition is formulated in a lyophilized form.

## Patentansprüche

1. Pharmazeutische Komposition, **dadurch gekennzeichnet, dass** sie S-Nitrosoglutathion (GSNO) und ein Polysacharid zusammen mit Poly(vinylalkohol) (PVA) und Polyethylenglykol (PEG) Polymeren und einem oder mehreren pharmazeutisch annehmbaren Zusatzstoff(en) enthält.

2. Pharmazeutische Komposition gemäß Anspruch 1, **dadurch gekennzeichnet, dass** das Polysacharid Chitosan ist.

3. Pharmazeutische Komposition gemäß Anspruch 1 oder 2, **dadurch gekennzeichnet, dass** die Komposition ein wässriges Gel ist.

4. Pharmazeutische Komposition gemäß einem der Patentansprüche 1 bis 3, **dadurch gekennzeichnet, dass** die Komposition in lyophilisierter Form formuliert ist.

## Revendications

1. Composition pharmaceutique **caractérisée en ce qu'**elle contient du S-nitrosoglutathione (GSNO) et un polysaccharide, de même que des polymères de poly(vinylalcool) (PVA) et de polyéthylèneglycol (PEG) ainsi qu'un ou plusieurs additif(s) acceptable(s) du point de vue pharmaceutique.

2. Composition pharmaceutique selon la revendication 1, **caractérisée en ce que** le polysaccharide est du chitosan.

3. Composition pharmaceutique selon la revendication 1 ou 2, **caractérisée en ce que** la composition est un gel aqueux.

4. Composition pharmaceutique selon l'une quelconque des revendications 1-3, **caractérisée en ce que** la composition est formulée sous forme lyophilisée.
